# EUROPEAN PATENT APPLICATION

(11) **EP 1 062 907 A1**
(43) Date of publication of application: **27.12.2000**
(21) Application number: 99305008.7
(22) Date of filing: 25.06.1999
(51) Int. Cl.: A61B 5/00

(54) **System for aiding remote diagnosis**

(71) Applicant: Fairfield Imaging Ltd., Turnbridge Wells, Kent TN1 1NU (GB)
(72) Inventor: SYMES, Clive, c/o Fairfield Imaging Ltd., Kent TN1 1NU (GB); CLINCH, Noah, c/o Fairfield Imaging Ltd., Kent TN1 1NU (GB)
(74) Representative: Haley, Stephen

(57) **Abstract**

A system provides visual data to a clinician. The system comprises means for obtaining an image of a site on a patient to be viewed by a clinician, and means for obtaining patient identifying data. Means for storing the image data, together with the data identifying the patient, and means for transmitting the stored image and patient data to a remote site are provided. There is also means for receiving and storing the image and patient data at the remote site for subsequent viewing by the clinician. The image may be a still or video image.

A corresponding method of operating such a system is also provided. Such a method may be employed in the detection of cervical cancer.

## Description

This invention relates to a system for aiding remote diagnosis.

There are many areas of medicine in which diagnosis of a particular ailment requires a blood or tissue sample to be obtained from a patient for an initial analysis, and for which the patient is then assessed by an expert consultant in the particular field if the initial analysis shows cause for concern. One example is the screening of cervical cancer. After an initial smear test, the smear being obtained by a local nurse or doctor, any patient considered to have an abnormal smear is referred to a consultant.

There are a number of problems associated with such an approach to diagnosis. Firstly, it may be that the consultant is based a considerable distance from the patient, meaning that the patient has to travel considerable distances to see the consultant. Secondly, a large proportion of the consultant's time may be taken up not in actual examination of the patient but in preparation for examination and reporting paperwork, resulting in the consultant having a backlog of patients. An additional problem is that information relating to the patient has to be transferred from the initial inspecting doctor or nurse to the consultant and back therefrom. This results in delays occurring and in the occurrence of lost paperwork. In addition, the whole process is extremely worrying for the patient. Concerns are naturally raised when the patient has to be called to a consultant, and the concerns can be compounded if a long wait is required to visit the consultant.

In many cases the concerns are eventually unfounded because of the need to be overly cautious in classifying the original test sample, such as the smear as being abnormal. This is a particularly significant problem. Traditionally clinicians screening samples have been extremely cautious during screening in order to avoid false negative diagnosis. Because of this a burden is placed on clinical consultants by hundreds of thousands of "borderline" patients, who in the vast majority turn out to be healthy.

The present invention seeks to provide a system to overcome the problems associated with this process.

According to the present invention there is provided a system for providing visual data to a clinician, the system comprising:
means for obtaining an image of a site on a patient to be viewed by a clinician;
means for obtaining patient identifying data;
means for storing the image data, together with the data identifying the patient;
means for transmitting the stored image and patient data to a remote site; and
means for receiving and storing the image and patient data at the remote site for subsequent viewing by the clinician.

The system may comprise means for obtaining video image data and means for compressing the video image data prior to storage. In such a case the storage may be digital and on computer, so that video images can be transmitted.

The means for obtaining and storing patient identifying data may further comprise means for storing other patient-related data.

The system may comprise means for transmitting plural sets of stored image and patient identifying data in a single transmission.

The means for storing the received image and patient data at the remote site may also comprise means for transmitting back diagnosis data from the clinician. Such means may include the provision of means for providing a list of diagnostic options from which the clinician can choose.

A corresponding method of operating such a system is also provided. Such a method may be employed in the detection of cervical cancer.

The system and the method of the present invention make it possible for relatively unskilled staff to be able to obtain the relevant image data necessary to make the diagnosis of the illness of concern. The image obtaining means, which may be a digital still or video camera and appropriate optical equipment (where required) can be at the patient's local surgery and is relatively inexpensive. This means that the patient does not have to travel large distances for the appropriate viewing, and can be viewed in a surround familiar to them. The storing of collected image data and patient identifying data together ensures that errors in collating the two sets of data cannot occur. Furthermore, storage of the data means that image data from various patients can be obtained throughout a working day and then transmitted at a time which is convenient and cost effective. Likewise, storage of relevant information at the receiving end of the system means that it can retrieved at any time convenient to the consultant. It also means that the data can be readily associated with any diagnostic information that the clinician decides to input, and can easily be transmitted back to the viewing site at a time which is also convenient.

The present invention has significant advantages. In particular, most patients for a program employing the invention will no longer need to enter a waiting list in order to be examined by a specialist consultant in a distant center. Instead, a trained nurse can obtain the necessary image at a local clinic. This will reduce considerably waiting lists and introduce cost savings by avoiding unnecessary gynecological examinations. Furthermore, consultant gynecologists can devote more time to employing their specialist knowledge more efficiently to those patients who have real symptoms of the disease.

One example of the present invention will now be described with reference to the accompanying drawings, in which:
Fig. 1 is a schematic diagram of a system according to the present invention; and
Fig. 2 is a schematic block diagram showing the internal operation of features of the system of figure 1.

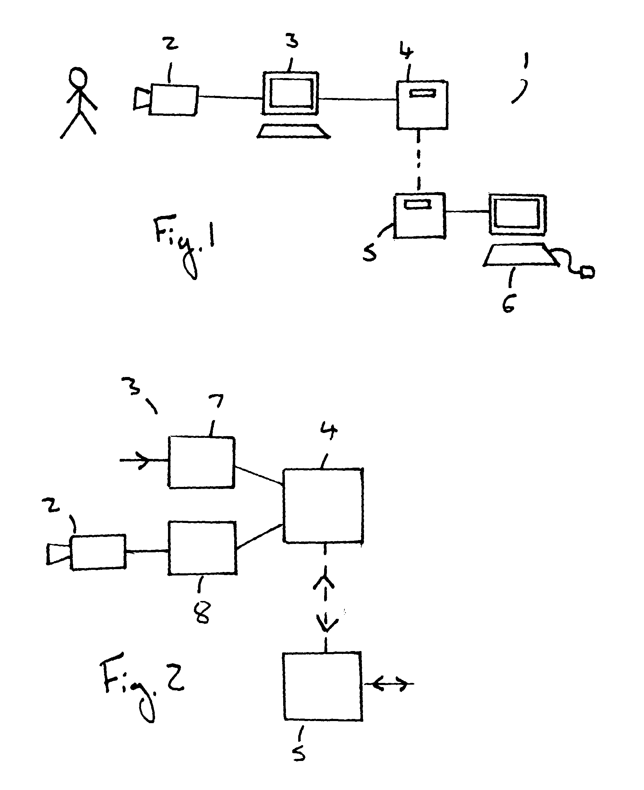

Referring to figure 1, a system 1 according to the present invention has an imaging device 2. The imaging device 2 may be a simple digital still or video camera, if it is to simply obtain images of a patient's skin, for example, or may be a colposcopy device with attached digital still or video camera if it is for a cervical inspection. It will be appreciated that this device can be adapted for other internal examinations if necessary. Imaging device 2 is attached to an image processing device 3, which may be an appropriately configured personal computer. The image processing device 3 also has means for receiving input from an operator in order to enable the operator to select appropriate images to be stored and also to enter patient's identifying data. The patient identifying data may simply be a name and date of birth, but can include information in relation to the medical history of the patient. The image processing means 3 compresses the retrieved and select image data and forwards it, with the patient identifying data, for storage in a storage means 4. At a selected time of day the data stored in the data storage means 4, which may be from multiple patients, is transmitted to a remote location via a dedicated line or, preferably, via an Internet connection. The data is received at a second data storage device 5, which stores the received data. At a time convenient to a clinician at the remote location, the data stored at the second data storage means 5 can be retrieved for viewing via a display and processing apparatus 6, which can be an appropriately configured personal computer. The clinician can then input, if necessary, comments and diagnostic information, which can be re-stored in the second data storage means 5. If necessary, the appropriate data can then be transmitted back to the first data storage means 4 for review by an operator at the patient's viewing location, so that the appropriate advice can be forwarded to the patient.

Figure 2 shows some of the internal components of the system, with the image processing means 3 having two key components, a patient data input means 7, for receiving data from a keyboard, mouse or other data input device, and an image receiving, selecting and compressing means 8. This image compressing means 8 may employ JPEG or other known image compressing algorithms, to reduce the amount of data that is required to be stored on the first data storage means 4, enabling video images to be stored and transmitted if necessary without requiring expensive memory and transmission circuitry.

## Claims

1. A system for providing visual data to a clinician, the system comprising:
means for obtaining an image of a site on a patient to be viewed by a clinician;
means for obtaining patient identifying data;
means for storing the image data together with the data identifying the patient;
means for transmitting the stored image and patient data to a remote site; and
means for receiving and storing the image and patient data at the remote site for subsequent viewing by the clinician.

2. A system according to claim 1, wherein the image is in video image and further comprising means for obtaining video image data and means for compressing the video image data prior to storage.

3. A system according to claim 1 or claim 2, wherein the means for obtaining and storing patient identifying data further comprises means for storing other patient-related data.

4. A system according to any of claims 1 to 3, wherein the system comprises means for transmitting plural sets of stored image and patient identifying data in a single transmission.

5. A system according to any preceding claim, wherein the means for storing the received image and patient data at the remote site also comprises means for transmitting back diagnosis data from the clinician.

6. A system according to claim 5, wherein the means for storing the received image and patient data includes the provision of means for providing a list of diagnostic options from which the clinician can choose.

7. A method for providing visual data to a clinician, the method comprising the steps of:
obtaining an image of a site on a patient to be viewed by a clinician;
obtaining patient identifying data;
storing the image data together with the data identifying the patient;
transmitting the stored image and patient data to a remote site; and
receiving and storing the image and patient data at the remote site for subsequent viewing by the clinician.

8. A method according to claim 7, wherein the patient site is the cervix.
